# EUROPEAN PATENT SPECIFICATION

(11) **EP 2 286 743 B1**
(45) Date of publication and mention of the grant of the patent: **31.07.2013**
(21) Application number: 09010673.3
(22) Date of filing: 19.08.2009
(51) Int. Cl.: A61B 17/225, A61B 19/00

(54) **Lithotripsy apparatus**
Lithotrypsievorrichtung
Appareil de lithotritie

(43) Date of publication of application: 23.02.2011
(73) Proprietor: Dornier MedTech Systems GmbH, 82234 Wessling (DE)
(72) Inventor: Artmeier, Theo, 82194 Gröbenzell (DE)
(74) Representative: Grünecker, Kinkeldey, Stockmair & Schwanhäusser

(56) References cited:
- WO-A1-2005/082261
- DE-A1- 4 400 997
- US-A- 5 285 772

## Description

The present invention relates to a lithotripsy apparatus comprising a carrying apparatus on which a therapy head is provided, wherein the carrying apparatus can be positioned between an X-ray source and an X-ray receiver of an X-ray device.

Related in prior is disclosed in DE 44 00997 A1 (on which the preamble of claim 1 is based), WO 2005/082261 A1 and US 5,285,772 A.

DE 198 08 402 A1 and DE 298 24 080 U1 disclose a lithotripsy apparatus of this type with a lithotripsy device and an X-ray device that has a C-bow on which an X-ray source and an X-ray receiver are provided on opposite ends. The C-bow is pivotable about a horizontal axis. Within the C-bow, a carrying apparatus of the lithotripsy device is provided, having a C-like carrying arm. A therapy head is held by means of a slide so that the therapy head can be moved orbitally. The carrying arm is pivotable about the same horizontal axis as said C-bow so that a patient can be reached in an under-table position as well as in an over-table position without repositioning the patient.

Identifying the three space coordinates of a target to be treated is easily possible. A first X-ray image is made in a first pivot position of the C-bow, and a second X-ray image is made in a second pivot position. The three space coordinates are determined from the two-dimensional coordinates of the target in the two X-ray images and the angle between the two pivot positions of the C-bow. There are always enough suitable pivot positions of the C-bow for making the two X-ray images in which the X-rays go past the lithotripsy device.

Starting from such a lithotripsy apparatus, DE 20 2004 010 005 A1 suggests a lithotripsy apparatus in which a carrying arm of a lithotripsy device is not positioned in an inside of a C-bow of an X-ray device but axially offset beside the C-bow so that there is more space within the C-bow. A cantilever is provided for bridging the axial distance between the carrying arm and the C-bow so as to position a focus of a therapy head within a plane defined by the C-bow.

The C-bow of DE 20 2004 010 005 A1 is pivotable about a first horizontal axis extending within a plane defined by the C-bow. Additionally, the C-bow is orbitally moveable about a second horizontal axis extending across to said first horizontal axis. In order to make two X-ray images to determine the three space coordinates of a target within a patient to be treated, the C-bow can be pivoted from a first pivot position to a second pivot position either about the first or the second horizontal axis. In order to make it possible to conduct an inline locating in which a shockwave axis of the therapy head and a central beam of the X-ray device are coinciding, the therapy head comprises a central radiolucent region. Thus, the "view" of the X-ray device is the same as that of the therapy head so that the progress of the treatment of the target, such as the destruction of a kidney stone, can be watched in the view of the therapy head during the treatment.

It is the object of the present invention to provide a lithotripsy apparatus of the above-mentioned type that has a good functionality and allows a good localization of a target.

According to the present invention, the object is solved by means of a lithotripsy apparatus having the features of independent claim 1.

As the carrying apparatus is at least partially radiolucent, a localizing action by means of X-rays is possible even if the carrying apparatus is positioned between an X-ray source and an X-ray receiver of an X-ray device. Thus, if the carrying apparatus is positioned in the X-ray path and an X-ray image is desired, the carrying apparatus can be left in this position. On the other hand, this means that more relative positions between the carrying apparatus and an assembly of an X-ray source and an X-ray receiver can be chosen for a localization action compared to a carrying apparatus that is not radiolucent. There is more operational freedom.

Preferably, a carrying arm of the carrying apparatus may be radiolucent at least between a section at which the carrying arm is supported and a distal end section of the carrying arm. This means that the carrying arm is radiolucent in a substantial section of its longitudinal extension.

Favourably, at least a middle part between lateral sides of a carrying arm of the carrying apparatus is radiolucent. Accordingly, the carrying arm has at least a window-like portion that is radiolucent.

According to the invention, a carrying arm of the carrying apparatus may have two opposing lateral frame members that are connected by means of at least one radiolucent connecting element. This means that at least one radiolucent element is part of the construction of the carrying arm and has a share in the radiolucent portion of the carrying arm.

Advantageously, the connecting element may be capable of transferring tensile and/or pressure forces between the opposing frame members. This stabilizes the carrying arm against lateral forces.

In a preferred embodiment, the connecting element may be a sheet-like element. Accordingly, the connecting element extends continuously, which provides for a good capability of transferring tensile and/or pressure forces.

Particularly preferable, the connecting element may have a continuous surface-to-surface connection to at least one of the frame members. The surface-to-surface connection provides for a good transfer of tensile and/or pressure forces between the connecting element and said frame member and prevents the connecting element from creeping at the section where it is connected to the frame member by means of the continuous surface-to-surface connection.

Favourably, the connecting element may have on at least one side an angled rim. The angled rim stabilizes the connecting element against bending forces, and also contributes to stabilization against lateral forces.

Preferably, the angled rim may be inserted into a slot of one of the frame members, the width of the slot corresponding to the width of the angled rim. This stabilizes the rim against crimping which, in turn, stabilizes the connecting element against bending. Further, the angled rim and the slot help to define the relative positions of the connecting element and said frame member. This contributes to an easy assembly step.

Particularly advantageous, the rim may be fixed to one of the frame members by means of a continuous surface-to-surface connection. This provides for a good holding of the rim on said frame member and for a good transmission of forces. Further, the surface-to-surface connection prevents a creeping of the rim, which contributes to stabilization of the connecting element.

Particularly favourable, the connecting element may be made from fiber-reinforced plastic. The fibers increase the capability of the connecting element to transfer forces, especially tensile forces, and may contribute to stabilization of the connecting element against bending.

In a preferred embodiment, at least two connecting elements are provided in a distanced parallel arrangement. Hereby, the carrying arm is particularly stabilized against torsional loads.

Favourably, the therapy head may be held on a carrying arm of the carrying apparatus by means of a holding structure having an area that is radiolucent, said area coinciding at least partially with a radiolucent portion of the carrying arm. Accordingly, the X-rays can pass through the holding structure as well as the carrying arm.

Advantageously, said area may be a cutout. Thus, the holding structure defines a particular light-weight structure allowing radiation to pass through.

Particularly favourable, the cutout may have an opened end and be provided at an end of the holding structure. Hereby, the radiation path is maintained, i.e. not interrupted if the holding structure starts moving into the range of the radiation path.

Preferably, the lithotripsy apparatus may comprise an X-ray device having a C-bow on which an X-ray source and an X-ray receiver are provided in an opposing manner. The C-bow and the carrying arm may be relatively moveable with respect to each other.

An embodiment of the invention is shown in the drawings and is described below. In the drawings, the following is shown:
- Figure 1: is a front view of a lithotripsy apparatus according to the present invention in a first adjustment,
- Figure 2: is a side view of the lithotripsy apparatus shown in Figure 1,
- Figure 3: is a front view of the lithotripsy apparatus in a second adjustment,
- Figure 4: is a side view of the lithotripsy apparatus shown in Figure 3,
- Figure 5: is a front view of the lithotripsy apparatus in a third adjustment,
- Figure 6: is a side view of the lithotripsy apparatus shown in Figure 5,
- Figure 7: is a front view of the lithotripsy apparatus in a fourth adjustment,
- Figure 8: is a side view of the lithotripsy apparatus shown in Figure 7,
- Figure 9: is a front view of the lithotripsy apparatus in a fifth adjustment,
- Figure 10: is a side view of the lithotripsy apparatus shown in Figure 9,
- Figure 11: is a front view of the lithotripsy apparatus in a sixth adjustment,
- Figure 12: is a side view of the lithotripsy apparatus shown in Figure 11,
- Figure 13: is a first perspective view of a carrying apparatus of a lithotripsy device of the lithotripsy apparatus,
- Figure 14: is another perspective view of said carrying apparatus,
- Figure 15: is a front view of a carrying arm of said carrying apparatus,
- Figure 16: is a longitudinal sectional view of the carrying arm according to a line XVI- XVI in Figure 15, and
- Figure 17: is a cross-sectional view of the carrying arm according to a line XVII-XVII in Figure 16.

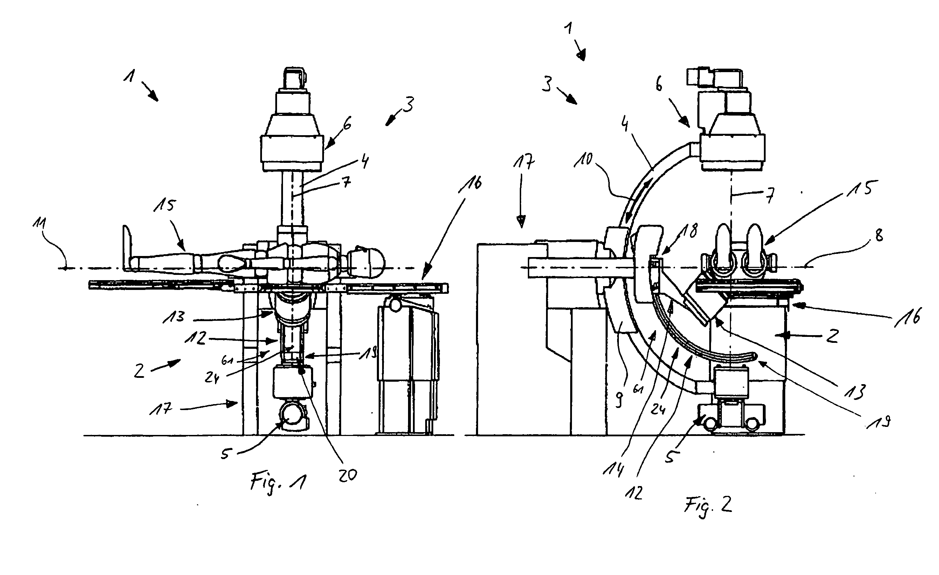

Figure 1 shows a front view of a lithotripsy apparatus 1 that is in accordance with the present invention. Figure 2 is a side view of this lithotripsy apparatus. The lithotripsy apparatus 1 comprises a lithotripsy device 2 and an X-ray device 3. The X-ray device 3 has a C-bow 4 to which an X-ray source 5 and an X-ray receiver 6 are fixed on opposing ends. A central X-ray beam 7 between X-ray source 5 and X-ray receiver 6 is indicated by means of a dash-dotted line.

The C-bow 4 has a C-like shape. In the present embodiment, it is an arc-like C-shape. In other embodiments, the C-bow can have other C-shapes, e.g. angular C-shapes.

The C-bow 4 is pivotable about a first horizontal axis 8 that intersects the central X-ray beam 7.

The C-bow 4 is slidably held by a guidance 9 so that the C-bow 4 can be orbitally moved as indicated by an arrow 10. In the present embodiment, the orbital movement is made about a second horizontal axis 11 that intersects the central X-ray beam 7 and the first horizontal axis 8.

The lithotripsy device 2 comprises a carrying apparatus 61 positioned within the C-bow 4. The carrying apparatus 61 has a carrying arm 12 and a holding structure 14. A therapy head 13 is held on the carrying arm 12 by means of the holding structure 14. The therapy head 13 is capable of generating shockwaves for treating a target within a patient 15, who is shown lying on a patient table 16. The carrying arm 12 is pivotable about a horizontal axis. In the present invention, the lithotripsy device 2 and X-ray device 3 are positioned with respect to each other such that this horizontal axis of the carrying arm 12 is identical with said first horizontal axis 8.

The holding structure 14 is a slide that can move along the carrying arm 12. The carrying arm 12 has a C-like shape that is an arc-like C-shape in the present embodiment. The therapy head 13 can be orbitally moved. The orbital movement is made about a horizontal axis. In the present invention, the lithotripsy device 2 and the X-ray device 3 are arranged with respect to each other such that this horizontal axis is identical with said second horizontal axis 11.

Accordingly, the lithotripsy device 2 and the X-ray device 3 have isocentrical kinematic adjustment systems, wherein the isocenter of the lithotripsy device 2 and the isocenter of the X-ray device 3 coincide. Further, a focus of the therapy head 13 coincides with these isocenters.

In the present embodiment, the lithotripsy device 2 and the X-ray device 3 have a common supporting base 17. However, it might be that the lithotripsy device 2 and the X-ray device 3 are separate devices, e.g. each having its own base. It might be that the lithotripsy device 2 and the X-ray device 3 can be moved away from one another so that each of them can be used at a different location. Such a separate lithotripsy device could be used with another X-ray device. Thus, there might be constructions in which the lithotripsy apparatus does not comprise an X-ray device.

The carrying apparatus 61 is at least partially constructed such that it is radiolucent, in particular for X-rays. Thus radiation can pass through the carrying apparatus if it, i.e. its radiolucent part, is positioned between the X-ray source 5 and the X-ray receiver 6. This means that a localizing action by means of the X-ray device 3 is possible, although the carrying apparatus 61 is positioned between the X-ray source 5 and the X-ray receiver 6.

In the present application, the term "radiolucent" is understood as subsuming cases in which there is a material through which radiation can pass, i.e. a through-passage for radiation is realized by means of radiolucent material, as well as cases in which the respective construction comprises a cutout serving as a through-passage for radiation. In other words, the carrying apparatus 61 is constructed such that it is through-passable for radiation, in particular for X-rays.

In the present embodiment, the carrying arm 12 is at least partially radiolucent, i.e. has a radiolucent portion 24 through which X-rays can pass if it is positioned between the X-ray source 5 and the X-ray receiver 6.

Preferably, the radiolucent portion 24 extends between a section 18 at which the carrying arm 12 is supported and a distal end section 19 of the carrying arm 12. This is the extension of the radiolucent portion in a lengthwise direction of the carrying arm. As to a widthwise direction, at least a middle part 20 between lateral sides of the carrying arm is radiolucent.

Thus, X-rays can pass through the carrying arm 12 being in the first adjustment shown in Figures 1 and 2 in which the carrying arm 12 is arranged parallel to the C-bow 4 in a so-called under table position, and is positioned between the X-ray source 5 and the X-ray receiver 6.

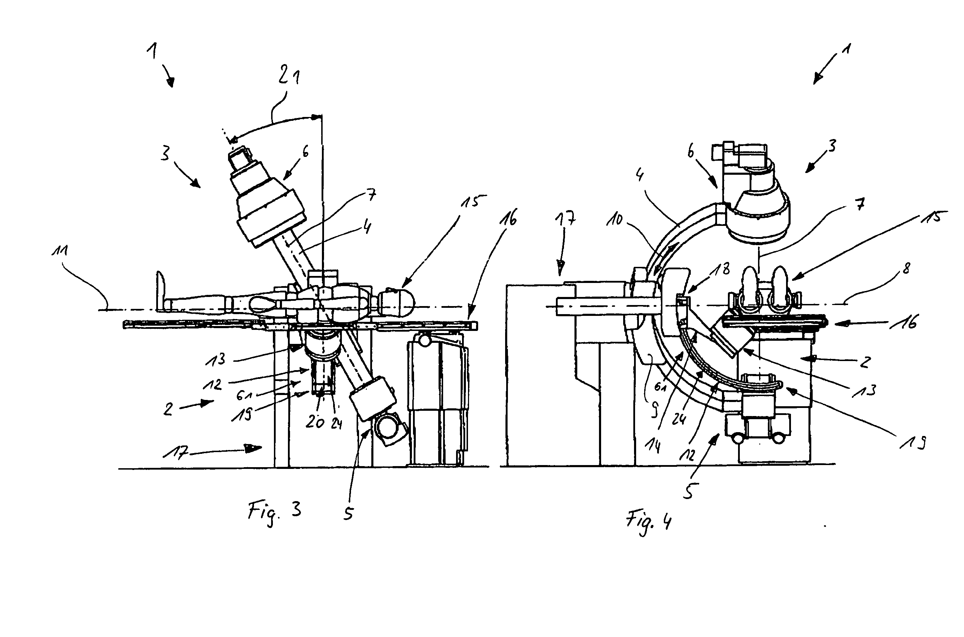

Figure 3 is a front view of the lithotripsy apparatus 1 in a second adjustment. In the view of Figure 3, the C-bow 4 is pivoted anti-clockwise about an angle 20 of approximately 30° relative to the carrying arm 12, compared to the first adjustment. The carrying arm 12 is not positioned between the X-ray source 5 and the X-ray receiver 6. Figure 4 is a side view of the lithotripsy apparatus 1 in the second adjustment.

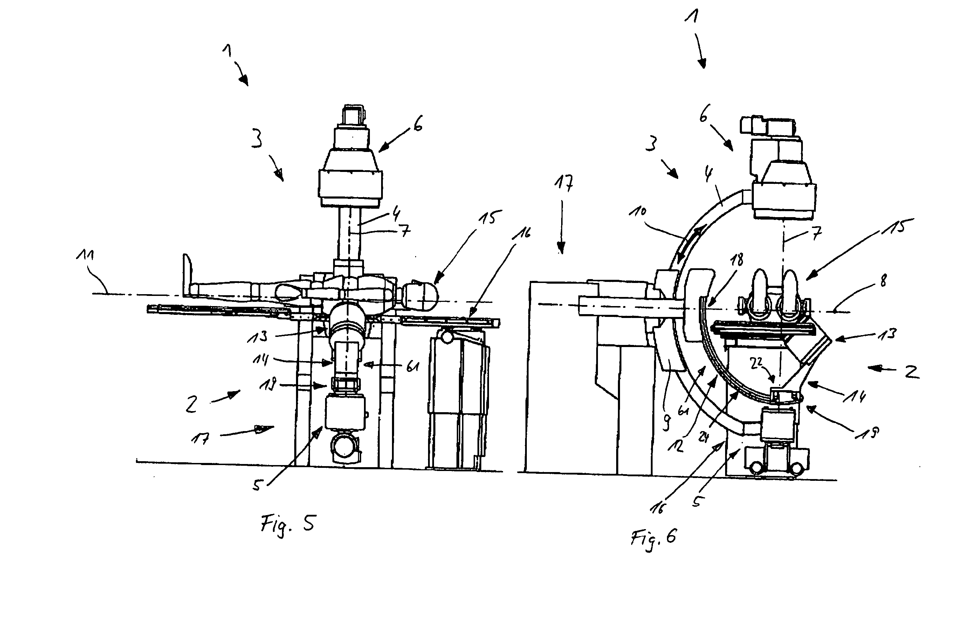

Figure 5 is a front view of the lithotripsy apparatus 1 in a third adjustment in which the carrying arm 12 is parallel to the C-bow and in the so-called under-table position. Compared to the first adjustment, the holding structure 14 is moved to the distal end section 19 of the carrying arm 12, so that the therapy head 13 is positioned to an opposing side of the patient 15. Figure 6 is a side view of the lithotripsy apparatus 1 in the third adjustment.

Also, the holding structure 14 defines an area which is radiolucent, in particular for X-rays. This area is a cutout portion 22 in the present embodiment, and will be discussed in greater detail later. The cutout portion 22 at least partially coincides with the radiolucent portion 24 of the carrying arm. Thus, a localizing action by means of the X-ray device 3 is possible, although the holding structure 14 is positioned between X-ray source 5 and X-ray receiver 6. Thus, the radiolucent part of the carrying apparatus 61 comprises the radiolucent portion 24 of the carrying arm 12 as well as the radiolucent area 22 of the holding structure 14.

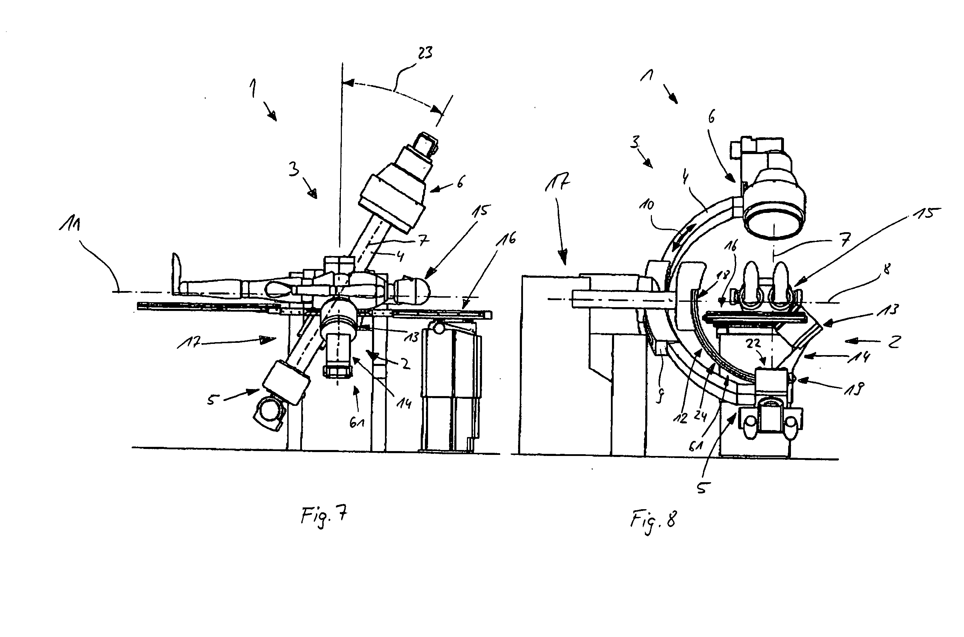

Figure 7 shows the lithotripsy apparatus 1 in a fourth adjustment in which, seen in the view of Figure 4 and compared to the third adjustment, the C-bow 4 is pivoted about an angle 23 of approximately 30° clockwise with respect to the carrying arm 12. That is, the carrying arm 12 and the holding structure 14 are not positioned between the X-ray source 5 and X-ray receiver 6. Figure 8 is a side view of the lithotripsy apparatus in the fourth adjustment.

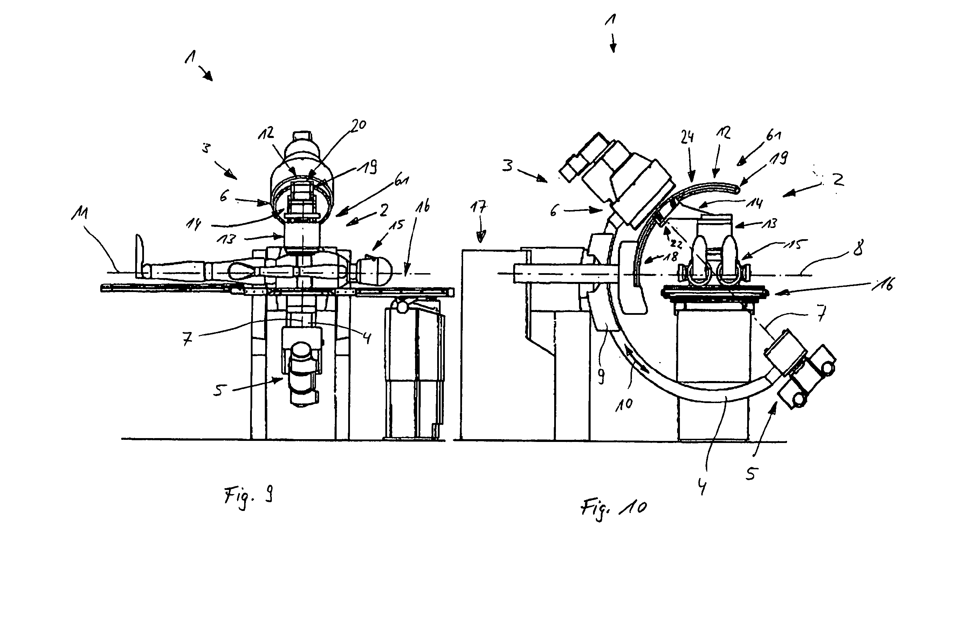

Figure 9 shows the lithotripsy apparatus 1 in a fifth adjustment in which the carrying arm 12 is parallel to the C-bow 4 and in a so-called over table position. The therapy head 13 is held in an approximately vertical position. As becomes clear from Figure 10, which is a side view of the lithotripsy apparatus 1 in the fifth adjustment, the C-bow is orbitally moved counterclockwise compared to the first adjustment, so that the angle between central X-ray beam 7 and the first horizontal axis 8 is approximately 45°. The carrying arm 12 and the holding structure 14 are positioned between X-ray source 5 and X-ray receiver 6. Nevertheless, a localizing action by means of the X-ray device 3 is possible, for the reasons explained already.

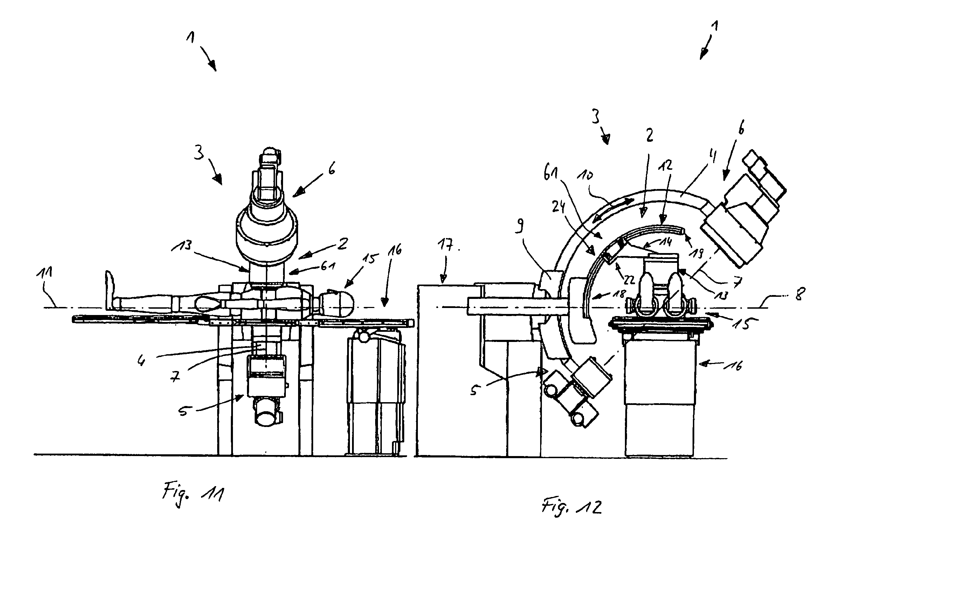

Figure 11 is a front view of the lithotripsy apparatus 1 in a sixth adjustment. Figure 12 shows a side view hereof. Compared to the fifth adjustment, the C-bow 4 is orbitally moved clockwise to the right about 90°, so that the angle between the central X-ray beam 7 and the first horizontal axis 8 is approximately 45°. Neither the carrying arm 12 nor the holding structure 14 is positioned between the X-ray source 5 and X-ray receiver 6.

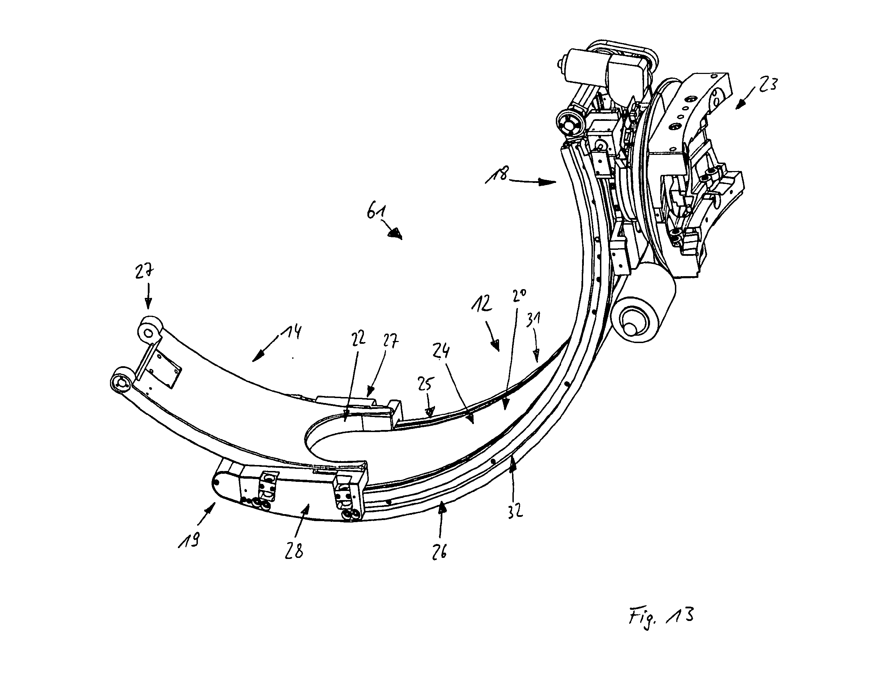

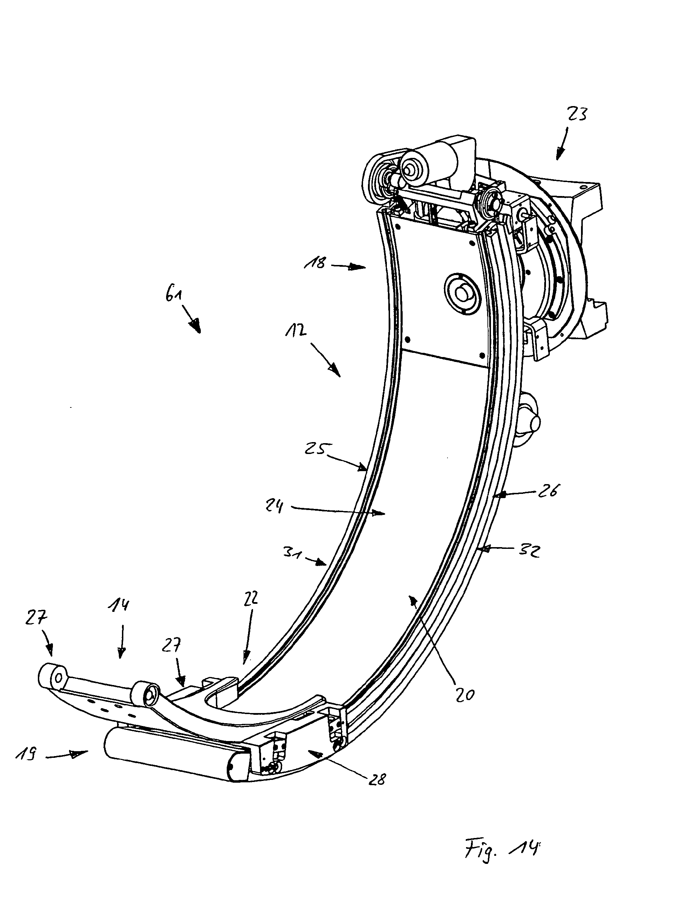

Figures 13 and 14 are different perspective views of the carrying apparatus 61 comprising the carrying arm 12, a supporting mechanism 23 supporting the carrying arm 12, and the holding structure 14. The radiolucent portion 24 of the carrying arm 12 extends between the section 18 at which the carrying arm 12 is supported by the supporting mechanism 23, the distal end section 19 of the carrying arm 12 and two opposing lateral frame members 25, 26. The particular construction of the carrying arm 12 will be described later.

The holding structure 14 has a distal end 27 to which the therapy head 13 will be fixed, and a proximal end 28 by means of which the holding structure 14 is held on the carrying arm 12. The cutout portion 22 is provided at the proximal end 28 and basically corresponds to the distance between the lateral frame members 25, 26, which is the width of the through-passable portion, i.e. radiolucent portion 24 of the carrying arm 12 of the present embodiment. The cutout portion 22 has an open end so that the proximal end of the holding structure 14 has a fork-like shape. In this embodiment, the cutout portion has a U-shape. However, other shapes for the cutout portion providing a window for the localizing action of the X-ray device 3 are suitable as well.

The holding structure 14 is attached to lateral sides of the carrying arm 12 and comprises, on its lateral sides, slight extensions 27, 28, which cooperate with guiding profiles 31, 32 on the lateral outer sides of the lateral frame members 25, 26. As shown in Figures 13 and 14, as well as in Figures 2, 4, 6, 8, 10 and 12, the holding structure 14 is arranged in an angled manner, in particular at an arcuate angle, with respect to the carrying arm 12. Further, it has a basically arc-like shape, as shown in Figures 13 and 14.

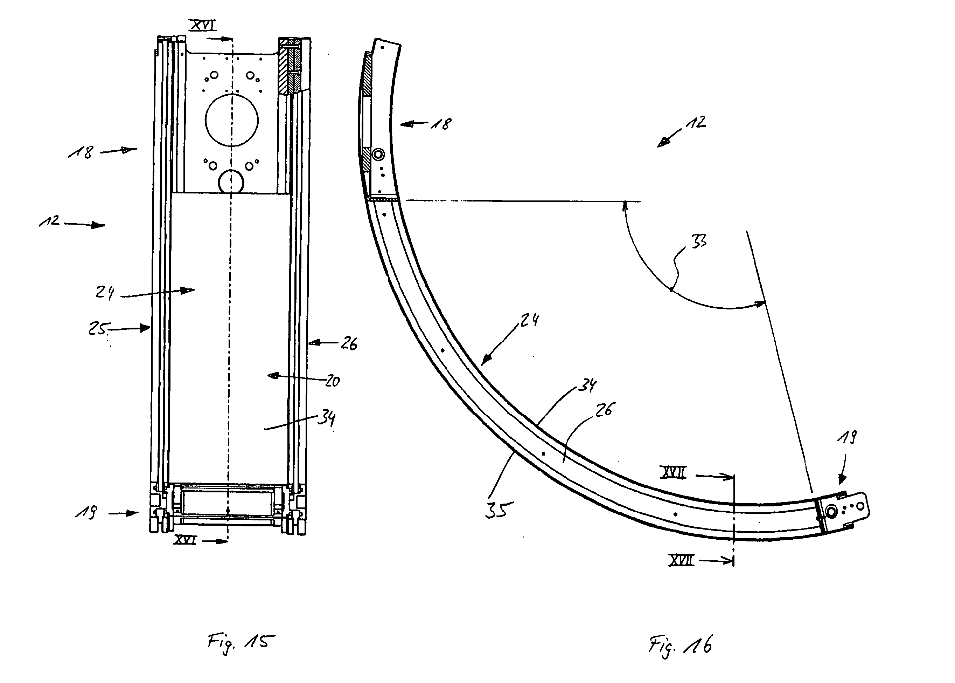

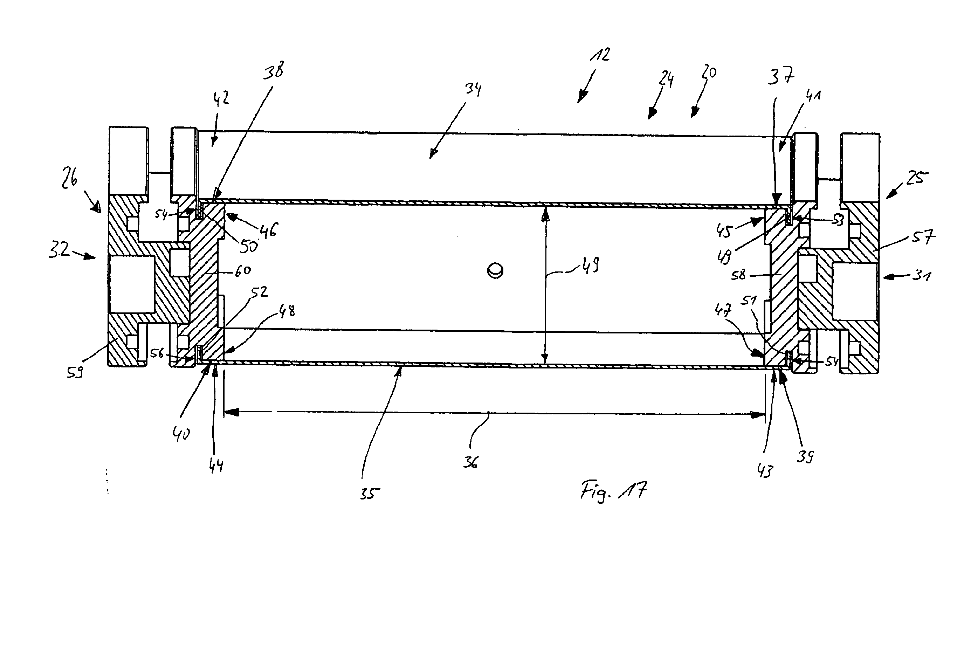

Figure 15 is a front view of a basic assembly of the carrying arm 12 with a partial free cut. Figure 16 is a longitudinal sectional view of said basic assembly along a line XVI-XVI in Figure 15. Figure 16 indicates the circumferential length 33 of the radiolucent portion 24. Figure 17 is a cross-sectional view of the carrying arm 12 along a line XVII-XVII in Figure 16.

As becomes clear from Figures 15 through 17, the lateral frame members 25, 26 are connected by means of a first and second connecting elements 34, 35. The first connecting element 34 is provided on an inside of the carrying arm 12 and the second connecting element 35 on an outside. Both connecting elements are made from radiolucent material, for instance, from plastic, and preferably from fiber-reinforced plastic. Thus, X-rays can pass through both connecting members 34, 35.

Each lateral frame member 25, 26 is made from metal in the present embodiment. Accordingly, the width 36 of the through-passable or radiolucent portion 24 corresponds to the distance between the lateral frame members 25, 26. Alternatively, the lateral frame members 25, 26 could be made from radiolucent material, so that the radiolucent portion of the carrying arm would be as wide as the carrying arm.

Each frame member 25, 26 is an assembly of at least two parts 57, 58, 59, 60.

The connecting elements 34, 35 are sheet-like or plate-like members that correspond to the shape in which the carrying arm 12 extends lengthwise, i.e. are C-shaped.

The connecting elements 34, 35 are capable of transferring tensile and pressure forces between the opposing lateral frame members 25, 26 and the connection between the connecting elements 34, 35 and the lateral frame members 25, 26 is configured such that these forces can be transferred in a good manner. In particular, the connecting elements 34, 35 have continuous surface-to-surface connections 37, 38, 39, 40, wherein a corresponding lateral outer section 41, 42, 43, 44 of the connecting elements 34, 35 are fixed to corresponding inner portions 45, 46, 47, 48 of the lateral frame members 25, 26. Preferably, the surface-to-surface connections are made by continuous surface-to-surface gluing. A distance 49 between the parallel connecting elements 34, 35 is approximately as large as the cross-sectional height of the lateral frame members 25, 26. In the present embodiment, the distance 49 is slightly smaller than the maximum cross-sectional height of the frame members 26, 25. The explained construction provides a high stability for the carrying arm 12, especially against lateral and torsional forces. Nevertheless, the carrying arm 12 has a light-weight construction in which in the range of the radiolucent portion 24 the inside of the carrying arm can be hollow, as becomes clear from Figure 16 in connection with Figure 17.

Due to the fact that the first connecting element 34 is provided on the inside of the carrying arm 12 and the second connecting element 35 on the outside, the connecting elements 34, 35 have also a covering function.

Each of the connecting elements 34, 35 has on each lateral outer section 41, 42, 43, 44 an angled extension, namely an angled rim 49, 50, 51, 52 that extends in an angled manner with respect to the respective lateral outer section. In the present embodiment, the angle is approximately 90°, i.e. each rim 49, 50, 51, 52 extends in a radial direction of the C-shaped carrying arm 12. The rims 49, 50, 51, 52 stabilize the connecting elements 34, 35 in said radial direction as well as against lateral forces.

Each rim 49, 50, 51, 52 is accommodated by a correspondingly formed slot 53, 54, 55, 56 of the respective lateral frame member 25, 26. In particular, the width of each slot 53, 54, 55, 56 is such that the respective rim 49, 50, 51, 52 can just be inserted. The rims 49, 50, 51, 52 are fixed within their respective slot 53, 54, 55, 56 by means of a continuous surface-to-surface connection. In the present embodiment, the rims 49, 50, 51, 52 are continuously glued into the slots 53, 54, 55, 56.

When the carrying arm 12 is assembled, the rims 49, 50, 51, 52 are hooked into slots 53, 54, 55, 56 so that the desired widthwise relative position of the lateral frame members 25, 26 is easily achieved. The rims 49, 50, 51, 52 are stabilized against crimping. This stabilization is achieved by the width of the slots 53, 54, 55, 56 corresponding to the width of the rims 49, 50, 51, 52 and by the continuous surface-to-surface connection of the rims to the slots. As the rims are prevented from crimping, the stabilization provided by the rims for the connecting elements 34, 35 is significantly increased.

It is noted that stabilization of the rims against crimping can be achieved by a continuous surface-to-surface connection alone or by the feature that the width of the slots corresponds to the width of the rims alone.

## Claims

1. Lithotripsy apparatus (1) with a carrying apparatus (61) on which a therapy head (13) is provided, wherein the carrying apparatus (61) can be positioned between an X-ray source (5) and an X-ray receiver (6) of an X-ray device (3),
wherein the carrying apparatus (61) is at least partially radiolucent, wherein X-rays can pass through a radiolucent part (22, 24) of the carrying apparatus (61) when same is positioned between said X-ray source (5) and said X-ray receiver (6), the carrying apparatus (61) having a carrying arm (12) the carrying arm (12) of the carrying apparatus (61) having two opposing lateral frame members (25, 26) **characterized in that** the two opposing frame members are connected by means of at least one radiolucent connecting element (34, 35).

2. Lithotripsy apparatus (1) according to claim 1, wherein the carrying arm (12) of the carrying apparatus (61) is radiolucent at least between a section (18) at which the carrying arm (12) is supported and a distal end section (19) of the carrying arm (12).

3. Lithotripsy apparatus according to claim 1 or 2, wherein at least a middle part (20) between lateral sides of the carrying arm (12) of the carrying apparatus (61) is radiolucent.

4. Lithotripsy apparatus according to claim 1-3, wherein the connecting element (34, 35) is capable of transferring tensile and/or pressure forces between the opposing frame members (25, 26).

5. Lithotripsy apparatus according to claim 1-4, wherein the connecting element (34, 35) is a sheet-like element.

6. Lithotripsy apparatus according to at least one of claims 1 to 5, wherein the connecting element (34, 35) has a continuous surface-to-surface connection to at least one of the frame members (25, 26).

7. Lithotripsy apparatus according to at least one claims 1 to 6, wherein the connecting element (34, 35) has at least on one side an angled rim (49, 50, 51, 52).

8. Lithotripsy apparatus according to claim 7, wherein the angled rim (49, 50, 51, 52) is inserted into a slot (53, 54, 55, 56) of one of the frame members (25, 26), the width of the slot corresponding to the width of the angled rim.

9. Lithotripsy apparatus according to claim 7 or 8, wherein the rim (49, 50, 51, 52) is fixed to one of the frame members (25, 26) by means of a continuous surface-to-surface connection.

10. Lithotripsy apparatus according to at least one of claims 1 to 9, wherein the connecting element (34, 35) is made from fiber-reinforced plastic.

11. Lithotripsy apparatus according to at least one of claims 1 to 10, wherein at least two connecting elements (34, 35) are provided in a distanced parallel manner.

12. Lithotripsy apparatus according to at least one of claims 1 to 11, wherein the therapy head (13) is held on the carrying arm (12) of the carrying apparatus (61) by means of a holding structure (14) having an area that is radiolucent, said area (22) coinciding at least partially with a radiolucent portion (24) of the carrying arm (12).

13. Lithotripsy apparatus according to claim 12, wherein said area is a cutout (22).

14. Lithotripsy apparatus according to claim 12 or 13, wherein the cutout (22) has an open end and is provided at an end (28) of the holding structure (14).

## Patentansprüche

1. Lithotripsie-Vorrichtung (1) mit einer Tragevorrichtung (61), an der ein Therapiekopf (13) vorhanden ist, wobei die Tragevorrichtung (61) zwischen einer Röntgenstrahl-Quelle (5) und einem Röntgenstrahl-Empfänger (6) einer Röntgenstrahleinrichtung (3) positioniert werden kann,
die Tragevorrichtung (61) wenigstens teilweise röntgenstrahldurchlässig ist, Röntgenstrahlen durch einen röntgenstrahldurchlässigen Teil (22, 24) der Tragevorrichtung (61) hindurchtreten können, wenn dieser zwischen der Röntgenstrahl-Quelle (5) und dem Röntgenstrahl-Empfänger (6) positioniert ist, die Tragevorrichtung (61) einen Tragearm (12) aufweist und der Tragearm (12) der Tragevorrichtung (61) zwei einander gegenüberliegende seitliche Rahmenelemente (25, 26) aufweist, **dadurch gekennzeichnet, dass** die zwei einander gegenüberliegenden Rahmenelemente mittels wenigstens eines röntgenstrahldurchlässigen Verbindungselementes (34, 35) verbunden sind.

2. Lithotripsie-Vorrichtung (1) nach Anspruch 1, wobei der Tragearm (12) der Tragevorrichtung (61) wenigstens zwischen einem Abschnitt (18), an dem der Tragearm (12) gelagert ist, und einem entfernten Endabschnitt (19) des Tragearms (12) röntgenstrahldurchlässig ist.

3. Lithotripsie-Vorrichtung nach Anspruch 1 oder 2, wobei wenigstens ein Mittelteil (20) zwischen Längsseiten des Tragearms (12) der Tragevorrichtung (61) röntgenstrahldurchlässig ist.

4. Lithotripsie-Vorrichtung nach Anspruch 1-3, wobei das Verbindungselement (34, 35) in der Lage ist, Zug- und/oder Druckkräfte zwischen den einander gegenüberliegenden Rahmenelementen (25, 26) zu übertragen.

5. Lithotripsie-Vorrichtung nach Anspruch 1-4, wobei das Verbindungselement (34, 35) ein plattenartiges Element ist.

6. Lithotripsie-Vorrichtung nach wenigstens einem der Ansprüche 1 bis 5, wobei das Verbindungselement (34, 35) eine kontinuierliche Flächenverbindung zu wenigstens einem der Rahmenelemente (25, 26) hat.

7. Lithotripsie-Vorrichtung nach wenigstens einem der Ansprüche 1 bis 6, wobei das Verbindungselement (34, 35) an wenigstens einer Seite einen abgewinkelten Rand (49, 50, 51, 52) hat.

8. Lithotripsie-Vorrichtung nach Anspruch 7, wobei der abgewinkelte Rand (49, 50, 51, 52) in einen Schlitz (53, 54, 55, 56) eines der Rahmenelemente (25, 26) eingeführt ist, und die Breite des Schlitzes der Breite des abgewinkelten Randes entspricht.

9. Lithotripsie-Vorrichtung nach Anspruch 7 oder 8, wobei der Rand (49, 50, 51, 52) an einem der Rahmenelemente (25, 26) mittels einer durchgehenden Flächenverbindung befestigt ist.

10. Lithotripsie-Vorrichtung nach wenigstens einem der Ansprüche 1 bis 9, wobei das Verbindungselement (34, 35) aus faserverstärktem Kunststoff besteht.

11. Lithotripsie-Vorrichtung nach wenigstens einem der Ansprüche 1 bis 10, wobei wenigstens zwei Verbindungselemente (34, 35) parallel beabstandet vorhanden sind.

12. Lithotripsie-Vorrichtung nach wenigstens einem der Ansprüche 1 bis 11, wobei der Therapiekopf (13) an dem Tragearm (12) der Tragevorrichtung (61) mittels einer Haltestruktur (14) gehalten wird, die einen Bereich hat, der röntgenstrahldurchlässig ist, und der Bereich (12) wenigstens teilweise mit einem röntgenstrahldurchlässigen Abschnitt (24) des Tragearms (12) deckungsgleich ist.

13. Lithotripsie-Vorrichtung nach Anspruch 12, wobei der Bereich ein Ausschnitt (22) ist.

14. Lithotripsie-Vorrichtung nach Anspruch 12 oder 13, wobei der Ausschnitt (22) ein offenes Ende hat und an einem Ende (23) der Haltestruktur (14) vorhanden ist.

## Revendications

1. Appareil de lithotritie (1) comportant un appareil porteur (61) sur lequel est fournie une tête thérapeutique (13), l'appareil porteur (61) pouvant être positionné entre une source de rayons X (5) et un récepteur de rayons X (6) d'un dispositif à rayons X (3),
dans lequel l'appareil porteur (61) est au moins partiellement radio transparent, les rayons X pouvant passer à travers une partie (22, 24) radio transparente de l'appareil porteur (61) lorsque celui-ci est positionné entre ladite source de rayons X (5) et ledit récepteur de rayons X (6), l'appareil porteur (61) présentant un bras porteur (12), le bras porteur (12) de l'appareil porteur (61) présentant deux éléments formant cadre (25, 26) latéraux opposés, **caractérisé en ce que** les deux éléments opposés formant cadre sont raccordés au moyen d'au moins un élément de raccordement radio transparent (34, 35).

2. Appareil de lithotritie (1) selon la revendication 1, dans lequel le bras porteur (12) de l'appareil porteur (61) est radio transparent au moins entre une section (18) au niveau de laquelle le bras porteur (12) est supporté et une section d'extrémité (19) distale du bras porteur (12).

3. Appareil de lithotritie selon la revendication 1 ou 2, dans lequel au moins une partie médiane (20) entre des côtés latéraux du bras porteur (12) de l'appareil porteur (61) est radio transparente.

4. Appareil de lithotritie selon l'une quelconque des revendications 1 à 3, dans lequel l'élément de raccordement (34, 35) est capable de transférer des forces de tension et/ou de pression entre les éléments formant cadre (25, 26) opposés.

5. Appareil de lithotritie selon l'une quelconque des revendications 1 à 4, dans lequel l'élément de raccordement (34, 35) est un élément en forme de panneau.

6. Appareil de lithotritie selon l'une quelconque des revendications 1 à 5, dans lequel l'élément de raccordement (34, 35) présente un raccordement en continu surface à surface à au moins un des éléments formant cadre (25, 26).

7. Appareil de lithotritie selon l'une quelconque des revendications 1 à 6, dans lequel l'élément de raccordement (34, 35) présente un rebord angulaire (49, 50, 51, 52) sur au moins un côté.

8. Appareil de lithotritie selon la revendication 7, dans lequel le rebord angulaire (49, 50, 51, 52) est inséré dans une fente (53, 54, 55, 56) de l'un des éléments formant cadre (25, 26), la largeur de la fente correspondant à la largeur du rebord angulaire.

9. Appareil de lithotritie selon la revendication 7 ou 8, dans lequel le rebord (49, 50, 51, 52) est fixé à l'un des éléments formant cadre (25, 26) au moyen d'un raccordement continu surface à surface.

10. Appareil de lithotritie selon l'une quelconque des revendications 1 à 9, dans lequel l'élément de raccordement (34, 35) est réalisé à partir de plastique renforcé par des fibres.

11. Appareil de lithotritie selon l'une quelconque des revendications 1 à 10, dans lequel au moins deux éléments de raccordement (34, 35) sont fournis de manière parallèle espacée.

12. Appareil de lithotritie selon l'une quelconque des revendications 1 à 11, dans lequel la tête thérapeutique (13) est tenue sur le bras porteur (12) de l'appareil porteur (61) au moyen d'une structure de retenue (14) présentant une zone qui est radio transparente, ladite zone (22) coïncidant au moins partiellement avec une partie radio transparente (24) du bras porteur (12).

13. Appareil de lithotritie selon la revendication 12, dans lequel ladite zone est une découpure (22).

14. Appareil de lithotritie selon la revendication 12 ou 13, dans lequel la découpure (22) présente une extrémité ouverte et est fournie au niveau d'une extrémité (28) de la structure de retenue (14).
